# EUROPEAN PATENT APPLICATION

(11) **EP 1 614 357 A1**
(43) Date of publication of application: **11.01.2006**
(21) Application number: 04016287.7
(22) Date of filing: 10.07.2004
(51) Int. Cl.: A23L 1/30, A23L 1/09, A23L 1/307, A23L 1/308, A23L 1/054, A23K 1/165, A61K 31/702, A61K 31/733, A61K 31/7016, A61K 31/715, A61K 31/716

(54) **Dietary supplements comprising prebiotics and fatty acid**

(71) Applicant: Cognis IP Management GmbH, 40589 Düsseldorf (DE)
(72) Inventor: Rull Prous, Santiago, 08034 Barcelona (ES)

(57) **Abstract**

Suggested are new advantageous dietary supplements, comprising
(a) prebiotics and
(b) physiologically active fatty acids, their salts or their esters.

## Description

### Object of the invention

The present invention relates to the area of food additives and is directed to dietary supplements comprising prebiotics and physiologically active fatty acids, their salts or esters and their uses as substituents for probiotics and for making medicaments to improve the health status of the body.

### State of the art

Probiotics contain live bacteria and represent an important part of the complex world of foods that are good for health. Its the bacteria and the metabolites which they produce that give these products their health promoting properties. The best known example of a probiotic is yoghurt. The experimental data for yoghurt is still not as conclusive as one would like, however, human studies related to the consumption of dietary milk products show increased milk digestibility, quicker recovery from certain types of diarrhoea, enhanced immune function, relation in certain cancers, and possible lowering of blood cholesterol levels.

Bacteria found in products like yoghurt, kefir or fermented vegetables usually aren't found in the human intestine. In fact, the intestinal environment is often a hostile one for these foreign bacteria. Because of this, bacteria eaten in probiotic products don't colonise the intestine but are flushed through and eliminated from the body.

The bacteria living in the intestine make up a very large and very diverse population. The numbers of each kind of bacteria change depending on age, diet, health status, and use of drugs and supplements. The effects are linked to the ability of the bacteria to adhere to the intestinal wall and use the semi-digested food that it passing through the intestines. There are a lot of different health claims linked to the administration of probiotics to the human body which are reported in the literature, such as
- reduction *of Heliobacter pylori,*
- reduction of allergic symptoms,
- relief from constipation,
- relief from inflammatory bowel syndrome,
- beneficial effects on mineral metabolism, particularly bone density and stability (osteoporosis prevention),
- reduction of cholesterol and triacylglycerol plasma concentrations, and especially
- cancer prevention.

However, probiotics also show some disadvantages. For example, in case of inflammatory diseases of the intestine the mucosa epidermis becomes permeable for probiotic bacteria, so that they can enter into the serum. The same is true for elderly people. Also in case of a medicamentation with antibiotics the administration of probiotics is not suggested in order to allow the intestine flora de regenerate without being mixed up with probiotic bacteria which cannot settle in the intestine. Therefore, the object of the present invention has been to provide an alternative for the administration of probiotics to the human body providing the same or at least comparable health benefits without being linked with the disadvantages as described above.

### Description of the invention

The present invention claims dietary supplements, comprising
(a) prebiotics and
(b) physiologically active fatty acids, their salts or their esters.

Surprisingly it has been found that mixtures of prebiotics and said physiologically active fatty acids, salts or esters, particularly conjugated linoleic acid show similar behaviour like probiotic bacteria, like e.g. *Bifidobacterium breve, Bifidobacterium infantis, Bifidobacterium longum, Bifidobacterium adolescentis, Lactobacillus bulgaricus, Lactobacillus acidophilus, Lactobacillus casei, Lactobacillus plantarum, Streptococcus faecium,* or *Streptococcus thermophilus* with respect to the health claims linked to said probiotic micro-organisms.

For example, the mixtures according to the invention leads to a significant reduction in the formation of lithocholic acid and deoxcholic acid, which belong to the so-called secondary bile acid and are regarded to be responsible for the developing of cancer in the large intestine.

The mixtures of the present invention therefore are able to replace probiotics in food compositions, like e.g. yoghurt without loss of performance and without the disadvantages of the state of the art as outlined above. Therefore, the dietary supplement compositions according to the present invention or preferably substantially free of probiotic micro-organisms.

### Prebiotics

Prebiotics are defined as non-digestible food ingredients that may beneficially affect the host be selectively stimulating the growth and/or the activity of a limited number of bacteria in the colon. The following describes the various oligosaccharides which can be taken into account as suitable prebiotics (component a) :
- Fructooligosaccharides
   Fructooligosaccharides or FOS typically refer to short-chain oligosaccharides comprised of D-fructose and D-glucose, containing from three to five monosaccharide units. FOS, also called neosugar and short-chain FOS, are produced on a commercial scale from sucrose using a fungal fructosyltransferase enzyme. FOS are resistant to digestion in the upper gastrointestinal tract. They act to stimulate the growth of *Bifidobacterium* species in the large intestine. FOS are marketed in the United States in combination with probiotic bacteria and in some functional food products.
- Inulins
   Inulins refer to a group of naturally-occurring fructose-containing oligosaccharides. Inulins belong to a class of carbohydrates known as fructans. They are derived from the roots of chicory *(Cichorium intybus)* and Jerusalem artichokes. Inulins are mainly comprised of fructose units and typically have a terminal glucose. The bond between fructose units in inulins is a beta-(2-1) glycosidic linkage. The average degree of polymerisation of inulins marketed as nutritional supplements is 10 to 12. Inulins stimulate the growth of *Bifidobacterium* species in the large intestine.
- Isomaltooligosaccharides
   Isomaltooligosaccharides comprise a mixture of alpha-D-linked glucose oligomers, including isomaltose, panose, isomaltotetraose, isomaltopentaose, nigerose, kojibiose, isopanose and higher branched oligosaccharides. Isomaltooligosaccharides are produced by various enzymatic processes. They act to stimulate the growth of *Bifidobacterium* species and *Lactobacillus* species in the large intestine. Isomalto oligosaccharides are marketed in Japan as dietary supplements and in functional foods. They are being developed in the United States for similar uses.
- Lactilol
   Lactilol is a disaccharide analogue of lactulose. Its pharmaceutical use is in the treatment of constipation and hepatic encephalopathy. Lactilol is also used in Japan as a prebiotic. It is resistant to digestion in the upper gastrointestinal tract and it is fermented by a limited number of colonic bacteria, resulting in an increase in the biomass of bifidobacteria and lactobacilli in the colon. Lactilol is known chemically as 4-0-(beta-D-galactopyranosyl)-D-glucitol. Lactilol is not approved for the treatment of hepatic encephalopathy or constipation in the U.S., and its use as a prebiotic is considered experimental. Lactilol is used in Europe as a food sweetener.
- Lactosucrose
   Lactosucrose is a trisaccharide comprised of D-galactose, D-glucose and D-fructose. Lactosucrose is produced enzymatically by the enzymatic transfer of the galactosyl residue in lactose to sucrose. Lactosucrose is resistant to digestion in the stomach and small intestine. It is selectively utilized by intestinal *Bifidobacterium* species resulting in significant induction of growth of these bacteria in the colon. Therefore, under physiological conditions, lactosucrose acts on the intestinal microflora as a growth factor for *Bifidobacterium* species. Lactosucrose is also known as 4G-beta-D-galactosylsucrose. It is widely used in Japan as a dietary supplement and in functional foods, including yoghurt. Lactosucrose is being developed in the United States for similar uses.
- Lactulose
   Lactulose is a semi-synthetic disaccharide comprised of the sugars D-lactose and D-fructose. The sugars are joined by a beta-glycosidic linkage, making it resistant to hydrolysis by human digestive enzymes. Lactulose is, however, fermented by a limited number of colonic bacteria. This can lead to changes in the colonic ecosystem in favour of bacteria, such as lactobacilli and bifidobacteria, which may confer some health benefits. Lactulose is a prescription drug in the United States for the treatment of constipation and hepatic encephalopathy. It is marketed in Japan for use as a dietary supplement and in functional foods. Its use in the United States as a prebiotic substance is still experimental.
- Pyrodextrins
   Pyrodextrins comprise a mixture of glucose-containing oligosaccharides that is derived from the hydrolysis of starch. Pyrodextrins have been found to promote the proliferation of *Bifidobacterium* species in the large intestine. They are resistant to digestion in the upper gastrointestinal tract. Pyrodextrins are being developed for the nutritional supplement market place.
- Soy oligosaccharides
   Soy oligosaccharides refer to oligosaccharides found in soybeans and also in other beans and peas. The two principal soy oligosaccharides are the trisaccharide raffinose and the tetrasaccharide stachyose. Raffinose comprises one molecule each of D-galactose, D-glucose and D-fructose. Stachyose consists of two molecules of D-galactose, one molecule of D-glucose and one molecule of D-fructose. Soy oligosaccharides act to stimulate the growth of *Bifidobacterium* species in the large intestine. They are marketed in Japan as dietary supplements and in functional foods. They are being developed in the United States for similar uses.
- Transgalactooligosaccharides
   Transgalactooligosaccharides (TOS) are a mixture of oligosaccharides consisting of D-glucose and D-galactose. TOS are produced from D-lactose via the action of the enzyme beta-galactosidase obtained from *Aspergillus oryzae*. TOS are resistant to digestion in the upper gastrointestinal tract and stimulate the growth of bifidobacteria in the large intestine. TOS are marketed in Japan and Europe as dietary supplements and are used in functional foods. They are being developed for similar use in the United States.
- Xylooligosaccharides
   Xylooligosaccharides are comprised of oligosaccharides containing beta (1→ 4) linked xylose residues. The degree of polymerisation of xylooligosaccharides is from two to four. Xylo oligosaccharides are obtained by enzymatic hydrolysis of the polysaccharide xylan. They are marketed in Japan as prebiotics and are being developed for similar use in the United States.
- Biopolymers
   Suitable biopolymers like e.g. beta-glucans include those originating from plants including cereals such as oats and barley, fungi, yeast, and bacteria. In addition, microbial cell wall preparations and whole cells rich in beta glucans are also suitable sources for beta glucan preparations useful for the present invention. Monomer residues in glucans can have 1-3 and 1-4, or 1-3 and 1-6 linkages (that is the monomer units are joined through 1,3, 1,4 or 1,6 bonds) and the percent of each type can vary. Preferably, beta glucans derived from yeast, particularly from *Saccharomyces,* preferably *Saccharomyces cerevisiae*, are used for the present invention. It will be appreciated, however, that other beta glucans would also be suitable. Further examples for suitable biopolymers are chitin and its derivatives, preferably oligoglucosamin and chitosan which represents a typical hydrocolloid. 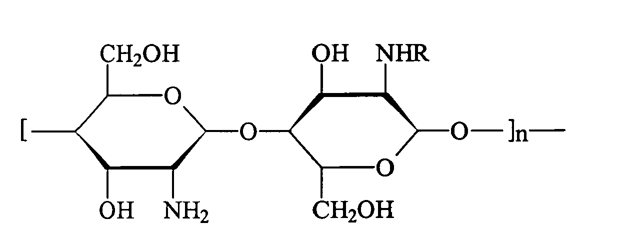
   Chitosan is obtained by deacetylisation of chitin and shows molecular weights in the range of 50.000 up to 2.000.000.

### Physiologically active fatty acids, their salts and their esters

A common criteria for fatty acids with physiological activity, which represent component (b), is a fat chain having a sufficient number of carbon atoms providing a lipophilic behaviour that allows the molecule pass through the gastrointestinal tract of the body and a sufficient number of double bonds. Therefore, said fatty acids usually comprise 18 to 26 carbon atoms and 2 to 6 double bonds.

In a first embodiment of the present invention conjugated linoleic acid (CLA) or its alkaline or alkaline earth salts and esters, preferably their calcium salts and their esters with lower aliphatic alcohols having 1 to 4 carbon atoms - or their glycerides, specially their triglycerides come into account. Conjugated linoleic acid (CLA) represents a commercially available product which usually is obtained by base-catalysed isomerisation of sunflower oil or their respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view the use of the *cis*-9,*trans*-11 isomer according to the present invention is of special importance having at least 30, preferably at least 50 and most preferably at least 80 % b.w. of said *cis*-9,*trans*-11 isomer - calculated on the total CLA content of the crude mixture. In addition, it has been found advantageous if the content of the *trans*-10,*cis*-12 isomer is at most 45, preferably at most 10 % b.w. and most preferably is less than 1 % b.w., and the sum of 8,10-, 11,13- and *trans,trans*-isomers in total is less than 1 % b.w. - again calculated on the total CLA content. Such products can be found in the market for example under the trademark Tonalin® CLA-80 (Cognis).

In a second embodiment also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example via transesterification of fish oils, followed by urea precipitation of the alkyl esters thus obtained and a final extraction using non-polar solvents as described in the German patent **DE 3926658 C2** (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found in the market under the trademark Omacor® (Pronova).

In a third embodiment also linoleic acid, vaccinic acid (trans 11-octadecenoic acid) or cishexadecenoic acid (obtained for example from the plant *Thunbergia alata*) can be used.

In addition said physiologically active fatty acid esters can not only be used in form of their lower alkyl esters or glycerides, an additional well preferred embodiment of the present invention relates to compositions comprising esters of said fatty acids with sterols. Like glycerides sterol esters are easily resorbed and splitted by the human body, however, a significant advantage comes from the fact that the cleavage of the ester bond releases a second molecule with health promoting properties. To avoid unclearities, the phrases "sterol", "stanol" and "sterin" shall be used as synonyms defining steroids showing a single hydroxyl group linked to the C-3. In addition sterols, which consist of 27 to 30 carbon atoms, may show a double bond, preferably in 5/6 position. According to the present invention esters of CLA or omega-3 fatty acids with β-sitosterol or its hydrogenation product β-sitostanol are preferred.

### Dietary supplements

The oral and/or topical compositions according to the present invention may comprise the prebiotics and the fatty acids in a weight ratio of 99 to 1 to 50 : 50 and more particularly 95 : 10 to 75 : 25. The highest synergistic effects, however, are observed at ratios of 92 : 8 to 80 : 20. In general, the compositions can be used in a concentration of up to about 90, particularly 10 to 75 and more particularly 25 to 50 % b.w. - calculated on the final food composition.

The compositions may further comprise certain plant extracts, like extracts of *Camellia sinensis* (Green tea) or *Olea europensis* (Olive tree) which are rich in actives like polyphenols, oleuropein and hydroxtyrosol in amounts of typically 1 to 10 and particularly 2 to 5 % b.w.

In a special embodiment of the present invention said dietary supplements are macro- or micro-encapsulated. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers). The encapsulation of the compositions according to the present invention is preferred in case the active should be liberated at the same part of the intestine. Therefore, one skilled in the art can easily select the adequate encapsulation system by comparing the stability of the capsules under the pH-conditions of the respective part of the intestine.

### Industrial application

A final object of the present invention is related to the use of mixtures, comprising
(a) prebiotics and
(b) physiologically active fatty acids, their salts or their esters
   as substituents for probiotic micro-organisms, particularly in food compositions like yoghurt and for making a medicament for improving the status of the human body, for example with respect to
   - reduction *of Heliobacter pylon* infection,
   - reduction of allergic symptoms,
   - relief from constipation,
   - relief from inflammatory bowel syndrome and inflammatories of the intestine,
   - beneficial effects from mineral metabolism, particularly bone density and stability (osteoporosis prevention),
   - reduction of cholesterol and triacylglycerol plasma concentrations, and
   - cancer prevention.

### Examples

### Preparation example 1

Soy milk was added to 15-75 parts by volume of cow milk to make 100 parts of the mixture. The mixture was then pasteurised at about 90 °C for 15 seconds and then cooled and separated into three samples. The cooled, pasteurised mixtures are then inoculated with 3 to 5 percent by volume of
(a) a standard yoghurt culture,
(b) a probiotic yoghurt culture having 1:1 ratio of *Lactobacillus bulgaricus* and *Bifidobacterium adolescentis,* and
(c) a standard yoghurt mixture comprising a 9 : 1 mixture of inulin and CLA.

The incubation was carried out at about 42 °C. After two hours thickening occured. The fermentation was carried out for about 5.5 hours.

### Example 2

The average faecal excretion of lithocholic and deoxycholic acid (secondary bile acids which represent toxic metabolisation products of the human body) related to 100 g fat intake per day has been studied over a period of two weeks. Table 1 reflects the results after consummation of the three yoghurt compositions according to example 1:

**Table 1 Faecal excretion of bile acids (mg/d)**

| **Faecal excretion** | **Standard yoghurt** | **Probiotic yoghurt** | **Standard yoghurt + inulin/CLA mixture** |
|---|---|---|---|
| Lithocholic acid | 305 ± 100 | 200 ± 80 | 210 ± 80 |
| Deoxycholic acid | 580 ± 100 | 390 ± 100 | 420 ± 100 |

The results indicate that the use of mixtures of prebiotics and physiologically active fatty acids leads to a comparable decrease of secondary bile acids as probiotics.

## Claims

1. Dietary supplements, comprising
(a) prebiotics and
(b) physiologically active fatty acids, their salts or their esters.

2. Dietary supplements according to claim 1, **characterised in that** said prebiotics (component a) are selected from the group consisting of fructooligosaccharides, inulins, isomaltooligosaccharides, lactilol, lactosucrose, lactulose, pyrodextrins, soy oligosaccharides, transgalactooligosaccharides, xylooligosaccharides and biopolymers.

3. Dietary supplements according to claims 1 and/or 2, **characterised in that** said physiologically active fatty acids (component b) comprise 18 to 26 carbon atoms and 2 to 6 double bonds.

4. Dietary supplements according to any of the claims 1 to 3, **characterised in that** component (b) represents esters of said fatty acids with glycerol or sterol.

5. Dietary supplements according to any of the claims 1 to 4, **characterised in that** component (b) represents conjugated linoleic acid or omega-3 fatty acids.

6. Dietary supplements according to any of the claims 1 to 5, **characterised in that** they comprise component (a) and (b) in weight ratios of from 99 : 1 to 50 : 50.

7. Dietary supplements according to any of the claims 1 to 6, **characterised in that** said compositions are substantially free of probiotic micro-organisms.

8. Dietary supplements according to any of the claims 1 to 7, **characterised in that** said mixtures are macro- or micro-encapsulated.

9. Use of mixtures according to claim 1 as substituents for probiotic micro-organisms.

10. Use of mixtures according to claims 1 for making a medicament for the improvement of the health status of the human body.

11. Use of mixtures according to claims 1 for making a medicament for the reduction of *Heliobacter pylon* infection.

12. Use of mixtures according to claims 1 for making a medicament for the reduction of allergic symptoms.

13. Use of mixtures according to claims 1 for making a medicament for the relief from constipation.

14. Use of mixtures according to claims 1 for making a medicament for the relief from inflammatory bowel syndrome and inflammatories of the intestine.

15. Use of mixtures according to claims 1 for making a medicament for the prevention of osteoporosis.

16. Use of mixtures according to claims 1 for making a medicament for the reduction of cholesterol and triacylglycerol plasma concentrations.

17. Use of mixtures according to claims 1 for making a medicament for the prevention of cancer.
